# FASCICULE DE BREVET EUROPEEN

(11) **EP 2 160 985 B1**
(45) Date de publication et mention de la délivrance du brevet: **01.02.2012**
(21) Numéro de dépôt: 09179648.2
(22) Date de dépôt: 09.03.2004
(51) Int. Cl.: A61B 17/17

(54) **Canon de perçage pour le positionnement d'un implant glénoidien**
Bohrlehre für die Positionierung eines Gelenkpfannen-Implantates
Drill guide for positioning a glenoid implant

(30) Priorité: 10.03.2003 FR 0302936
(43) Date de publication de la demande: 10.03.2010
(62) Demande divisionnaire de: 04356033.3
(73) Titulaire: Tornier, 38330 Saint-Ismier (FR)
(72) Inventeur: Tornier, Alain, 38330, SAINT ISMIER (FR); Ekelund, Anders, 16761, BROMMA (SE); Comtat, Laurent, 38600, FONTAINE (FR)
(74) Mandataire: Grand, Guillaume

(56) Documents cités:
- US-A- 5 030 219
- US-A- 5 769 856
- US-A- 5 800 551
- US-B1- 6 379 386

## Description

La présente invention concerne un ancillaire de pose d'un implant glénoïdien.

Lors de l'implantation d'une prothèse d'épaule, il est parfois nécessaire de poser à l'extrémité articulaire de l'omoplate d'un patient, un implant glénoïdien qui, d'un côté, s'ancre rigidement dans l'omoplate et qui, de l'autre côté, délimite une surface articulaire de coopération avec la tête d'un implant huméral fixé à l'extrémité correspondante de l'humérus du patient. Pour ancrer fermement l'implant glénoïdien dans l'omoplate, cet implant comporte généralement une quille en saillie du reste de l'implant et destinée à être reçue et immobilisée dans un canal creusé à l'intérieur de l'omoplate à l'aide d'un organe approprié, notamment le forêt d'une perceuse à usage médical. Lors de l'application de cet organe sur l'omoplate, il est courant d'utiliser un ancillaire de guidage qui limite les risques de dérapage de la tige ou de la pointe de cet organe.

Le document US-B1-6,379,386 décrit de tels ancillaires de guidage. En particulier, en regard de ses figures 5 à 9, ce document décrit un ancillaire de guidage pour le foret d'une perceuse, comportant une platine destinée à venir en appui surfacique contre la glène de l'omoplate d'un patient et à l'intérieur de laquelle est formé un trou central de guidage du foret de perçage. Grâce à cet ancillaire, le chirurgien est à même de creuser une amorce d'un canal. Ce document décrit également, en regard de ses figures 15 à 17, un autre ancillaire comportant une platine d'appui sur l'omoplate, à l'intérieur de laquelle sont formés trois trous de guidages successifs d'un foret de perçage. La face de la platine tournée vers l'omoplate est pourvue d'une saillie adaptée pour venir se loger dans l'amorce préalablement creusée. Par applications successives de la perceuse au niveau de chacun des trois trous, le chirurgien dégage un volume de matière osseuse important et creuse ainsi un canal de réception suffisamment grand pour la quille de l'implant glénoïdien envisagé dans ce document.

Selon la morphologie du patient, l'état de l'omoplate sur laquelle un implant glénoïdien est destiné à être posé, les conditions opératoires et/ou la forme exacte de l'implant à poser, la direction longitudinale du canal de réception de la quille de l'implant est différente. Le chirurgien a généralement intérêt à creuser un canal dans la direction où l'omoplate du patient est la plus résistante, notamment dans une zone épaisse où l'os est abondant et sain.

Actuellement, la direction pour creuser ce canal est repérée sur l'omoplate soit de façon empirique par le chirurgien, soit, lorsque ce dernier utilise un ancillaire tel que celui décrit dans le document US-6,379,386 mentionné ci-dessus, arbitrairement imposée par la platine de l'ancillaire utilisé. Si le chirurgien tente d'appliquer l'organe de perçage suivant une direction choisie sans utiliser d'ancillaire de guidage, il court le risque de voir l'organe de perçage déraper et/ou creuser un canal dans une direction différente de celle qu'il visait, moins favorable pour la pose et la tenue ultérieure de l'implant glénoïdien.

Le but de la présente invention est de proposer un ancillaire de structure simple et qui permette d'imposer à l'organe de perçage, de façonnage ou analogue utilisé lors de la pose d'un implant glénoïdien, par exemple au foret d'une perceuse à usage médical, une direction d'application choisie par le chirurgien, le cas échéant en préopératoire, sur toute une gamme de directions envisageables, voire pour n'importe quelle direction anatomiquement envisageable.

A cet effet, l'invention a pour objet un ancillaire de pose d'un implant glénoïdien, tel que défini à la revendication 1.

Par sollicitation de ces moyens d'ajustement, le chirurgien est à même de disposer d'un guidage ajusté suivant la direction qu'il aura préalablement choisie, par exemple suite à l'analyse de coupes scanner de l'omoplate réalisées avant l'intervention chirurgicale. Lorsque ces moyens d'ajustement sont adaptés pour être reliés de façon amovible à la platine, le chirurgien peut juste avant l'opération, ou si nécessaire en per-opératoire, ajuster la direction de guidage imposée à l'organe de perçage utilisé.

D'autres caractéristiques de cet ancillaire, prises isolément ou selon toutes les combinaisons techniquement possibles, sont spécifiées aux revendications 2 à 9.

On propose également ici une méthode de pose d'un implant glénoïdien, dans laquelle :
- on dispose d'au moins une coupe scanner ou analogue de l'omoplate d'un patient,
- on détermine une direction préférentielle d'application sur l'omoplate d'un organe de perçage, de façonnage ou analogue,
- on utilise un ancillaire de guidage qui comporte, à la fois, une platine formant un guide directionnel pour ledit organe de perçage et des moyens d'ajustement de la direction du guide par rapport à la platine, lesquels moyens d'ajustement de l'ancillaire de guidage comportent un canon pourvu de plusieurs trous traversants rectilignes,
- on plaque une surface de la platine sur la glène de l'omoplate,
- on introduit l'organe de perçage dans le trou qui, parmi les trous traversants du canon, s'étend suivant une direction proche ou la direction la plus proche de la direction préférentielle, et
- on actionne l'organe de perçage de façon à agir sur l'omoplate suivant la direction préférentielle.

L'invention sera mieux comprise à la lecture de la description qui va suivre, donnée uniquement à titre d'exemple et faite en se référant aux dessins sur lesquels :
- la figure 1 est une vue en perspective d'un ancillaire selon l'invention ;
- la figure 2 est une vue en élévation, prise selon la flèche Il indiquée sur la figure 1, de l'ancillaire appliqué sur une omoplate d'un être humain ;
- la figure 3 est une vue en perspective en éclaté de l'ancillaire de la figure 1 ;
- les figures 4 et 5 sont des coupes selon les plans indiqués IV et V sur la figure 3 ;
- la figure 6 est une vue analogue à la figure 3 d'une variante de l'ancillaire de la figure 1 ; et
- la figure 7 est une vue en élévation prise selon la flèche VII indiquée sur la figure 6.

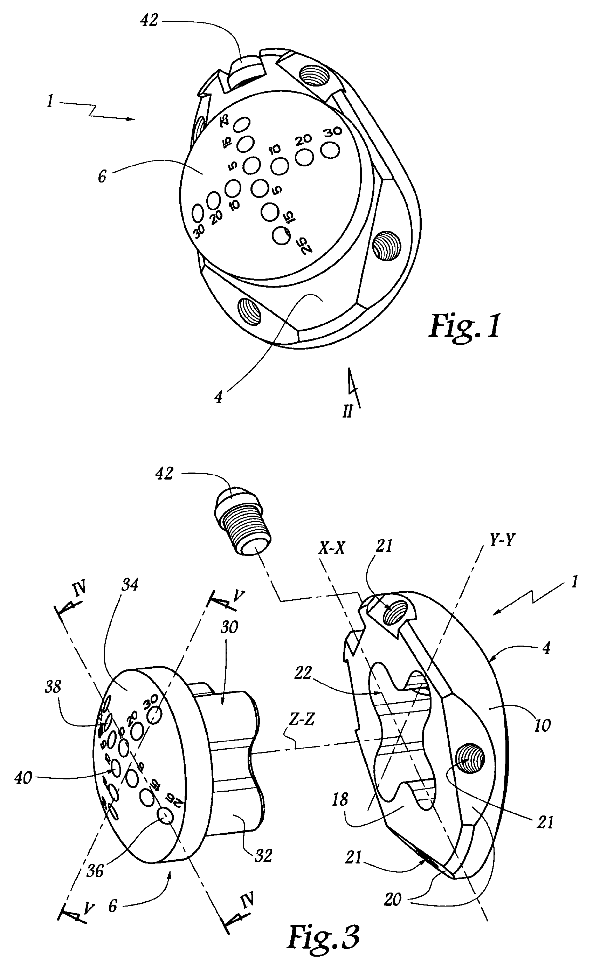

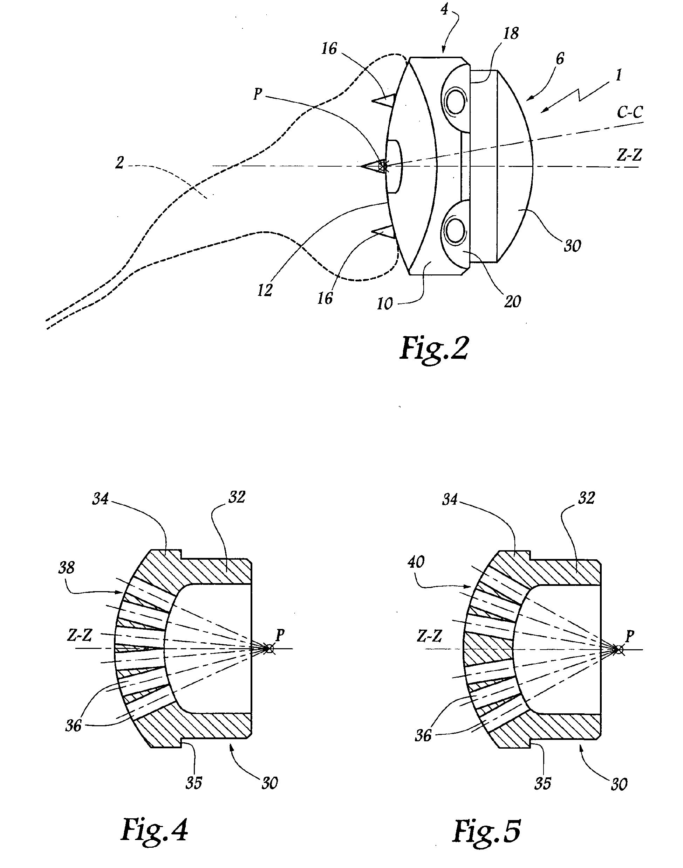

Sur la figure 1 est représenté un ancillaire 1 de pose d'un implant glénoïdien, adapté pour guider directionnellement le foret d'une perceuse à usage médical non représentée. Lors de la pose d'un implant glénoïdien sur l'omoplate d'un patient, en vue de former une prothèse d'épaule, le chirurgien doit creuser dans la glène de l'omoplate un canal de réception pour une quille de l'implant glénoïdien. En fonction de la morphologie du patient, de la géométrie de cette quille, de la pathologie ayant motivée la pose de l'implant, etc., la direction suivant laquelle ce canal est à former est différente. Pour déterminer la direction optimale de ce canal, le terme « optimal » signifiant ici « préférentiel » en considérant l'ensemble des paramètres médicaux portés à la connaissance du chirurgien, ce dernier dispose par exemple d'une série de coupes scanner de l'omoplate sur laquelle il est prévu de poser l'implant et détermine cette direction privilégiée. La localisation exacte du point d'application de l'organe de perçage est secondaire par rapport à la direction privilégiée, étant entendu que ce point d'application se situe dans une zone centrale de la cavité glénoïdienne. La figure 2 représente en pointillé une omoplate 2 d'un patient telle qu'elle apparaît au chirurgien par une coupe scanner. L'axe C-C indique la direction privilégiée choisie par le chirurgien comme étant la direction suivant laquelle l'organe de perçage devrait être appliqué.

A cet effet, l'ancillaire 1 comporte essentiellement une platine 4 sur laquelle sont rapportés des moyens 6 d'ajustement de la direction d'un guide de perçage par rapport à la platine.

Plus précisément, comme représenté sur les figures 1 à 5, la platine 4 est formée d'un corps métallique 10 présentant, suivant une direction notée Z-Z une dimension plus petite que sur les deux directions orthogonales restantes, indiquées par des axes X-X et Y-Y. Par la suite, les termes inférieur et supérieur seront orientés par rapport à l'axe X-X de sorte que, par exemple en regard de la figure 3, le terme supérieur correspond à la partie haute de cette figure, tandis que le terme inférieur correspond à la partie basse.

Le corps 10 de la platine 4 délimite une surface convexe 12 destinée à venir en appui surfacique contre l'omoplate 2, comme représenté sur la figure 2. Avantageusement, la surface 12 est pourvue de picots saillants 16 destinés à faciliter la retenue de la platine par rapport à l'omoplate comme il sera expliqué plus loin.

Le corps 10 délimite une surface 18 opposée à la surface 12, sensiblement plane et à partir de laquelle sont ménagées, aux quatre coins du corps 10, des surfaces biseautées 20. Dans ces surfaces 20 sont formés des évidements taraudés 21, adaptés pour recevoir des moyens (non représentés) de fixation amovible pour un manche de manipulation de la platine connu en soi.

Le corps 10 délimite intérieurement une ouverture traversante 22, d'axe central Z-Z et de profil cruciforme en coupe transversale. Plus précisément, l'ouverture 22 comporte deux paires de lobes diamétralement opposés, les lobes d'une même paire étant alignés respectivement suivant les directions X-X et Y-Y.

Les moyens d'ajustement 6 sont formés par un canon 30 représenté plus en détail sur les figures 3 à 5. Ce canon comporte un corps tubulaire 32 d'axe Z-Z et de profil extérieur sensiblement complémentaire du profil de l'ouverture traversante 22 du corps 10. Le corps 32 est fermé à une de ses extrémités par un fond globalement hémisphérique 34 qui forme, avec la surface extérieure du corps 32, un épaulement plan 35 radialement saillant par rapport à l'axe Z-Z.

A l'intérieur de ce fond 34 sont formés des trous traversants rectilignes 36 dont les axes respectifs sont sensiblement concourants en un point noté P appartenant à l'axe Z-Z. Ces trous 36 se répartissent en une première série 38 de trous qui s'étendent dans un premier plan contenant les axes X-X et Z-Z et correspondant sensiblement au plan de coupe de la figure 4, et une seconde série 40 de trous qui s'étendent dans un second plan contenant les axes Y-Y et Z-Z et correspondant au plan de coupe de la figure 5.

Dans chaque série 38, 40 de trous 36, l'écartement angulaire entre deux trous adjacents situés d'un même côté de l'axe Z-Z vaut sensiblement 10°. L'écartement angulaire entre l'axe Z-Z et l'un des deux trous 36 de la série 38 situés le plus au centre du couvercle 34 vaut environ 5°, tandis que le même écart angulaire pour les deux trous les plus au centre de la série 40 vaut environ 10°

Par coopération du corps tubulaire 32 avec l'ouverture traversante 22 de la platine 10, le canon 30 est à même d'être reçu et immobilisé par rapport à la platine 4, l'épaulement 35 se positionnant alors en butée contre la surface sensiblement plane 18 de la platine, comme représenté sur la figure 2. Le point P est alors inscrit sensiblement dans le prolongement géométrique de la face 12 destinée à venir en appui contre l'omoplate 2.

Dans la partie supérieure de la platine 4, une vis 42 est logée dans un perçage complémentaire s'étendant suivant la direction X-X et débouchant d'un côté sur le chant du corps 10 et de l'autre côté dans l'ouverture 22.

Cette vis permet de retenir suivant l'axe Z-Z le canon 30 dans l'ouverture 22.

L'utilisation de l'ancillaire 1 lors de la pose d'un implant glénoïdien pour prothèse d'épaule est la suivante :

Lorsque le chirurgien a déterminé, par exemple au moyen de coupes scanner de l'épaule d'un patient, la direction privilégiée C-C suivant laquelle il souhaite ancrer l'implant glénoïdien dans l'omoplate et donc appliquer un foret de perçage, il met en appui l'ancillaire 1 contre l'omoplate. Plus précisément, la surface 12 de la platine 4 est plaquée contre la glène osseuse de l'omoplate 2, comme représentée sur la figure 2. A cet effet, l'ancillaire 1 est avantageusement manipulé par un manche relié à la platine 10 au niveau des surfaces biseautées 20. Les picots 16 facilitent l'amarrage de la platine par rapport à l'omoplate.

Alors que le canon 30 est reçu et immobilisé dans l'ouverture 22 de la platine, le chirurgien sélectionne, parmi l'ensemble des trous 36 du canon, l'unique trou dont l'axe longitudinal est le plus proche de la direction préférentielle C-C qu'il a choisie. Si nécessaire, le chirurgien dégage le canon 30 de la platine, le fait pivoter d'un quart de tour puis le remet en place dans l'ouverture cruciforme 22, ce qui permet de disposer de directions de perçage ajustables avec un pas de 5° dans les deux plans contenant respectivement les axes X-X et Z-Z et les axes Y-Y et Z-Z.

Sur la figure 2, la direction choisie C-C forme, dans un plan contenant les axes Y-Y et Z-Z, un écart angulaire d'environ 15° par rapport à l'axe central de la cavité glénoïdienne, de sorte que le chirurgien va utiliser un seul des trous de la série 38 du canon.

Il introduit ensuite dans ce trou le foret d'une perceuse, afin de réaliser un perçage ou au moins une amorce de perçage dans la direction imposée par le trou. Si nécessaire, après avoir dégagé l'ancillaire 1, le chirurgien peut élargir l'orifice percé dans l'omoplate par un foret de plus grand diamètre ou un autre organe de façonnage. Il peut également utiliser une broche de fraisage qui, calée dans l'orifice percé, permet de ménager un plan de résection glénoïdien orthogonal à la direction C-C.

L'ancillaire 1 est ainsi d'utilisation facile et met rapidement à disposition du chirurgien un guide d'application d'un organe, notamment un guide de perçage, dont la direction est ajustée suivant la direction préférentielle que le chirurgien a choisie.

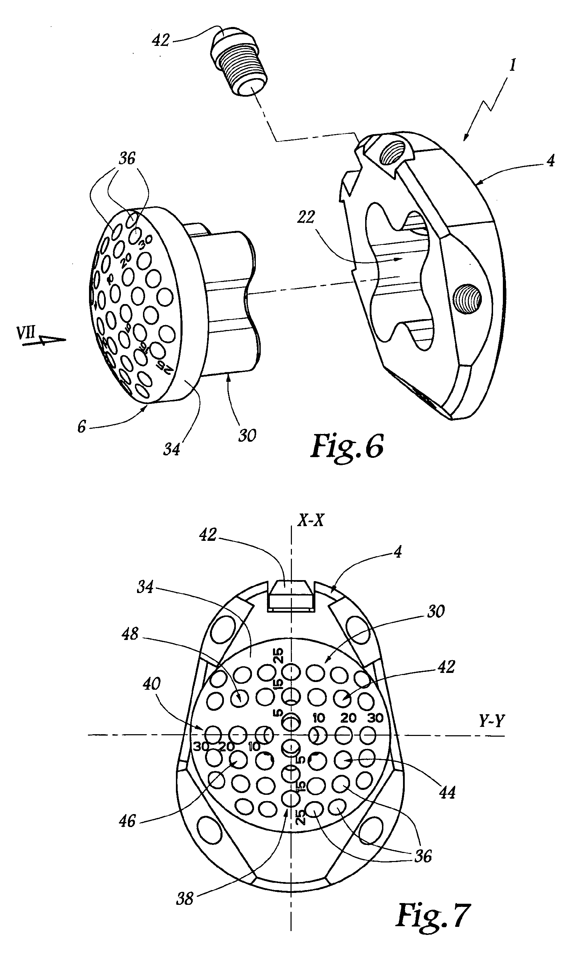

Sur les figures 6 et 7 est représentée une variante de l'ancillaire 1 qui se distingue de l'ancillaire des figures précédentes par le nombre et la disposition des trous de guidage 36 ménagés par le canon 30. En plus des séries de trous 38 et 40, des séries de trous 42, 44, 46 et 48 sont situés respectivement dans les quatre quadrants délimités par les séries de trous 38 et 40, en formant un quadrillage du fond 34 du canon. Cette variante met à la disposition du chirurgien plus de directions de guidage que l'ancillaire des figures 1 à 5.

Divers aménagements et variantes à l'ancillaire décrit ci-dessus sont en outre envisageables. A titre d'exemple, la face d'appui 12 est plane. De plus, la position du point P précité peut s'écarter quelque peu de celle décrite plus haut.

De même, l'ancillaire selon l'invention est à même de guider directionnellement tout organe de perçage, de façonnage ou analogue autre qu'un forêt de perçage, par exemple une broche à impacter ou un clou de marquage.

## Revendications

1. Ancillaire de pose d'un implant glénoïdien, comportant une platine (4) qui délimite une surface d'appui (12) destinée à venir s'appuyer contre la glène d'une omoplate (2) d'un patient et qui forme un guide directionnel pour un organe de perçage, de façonnage ou analogue, l'ancillaire comportant en outre des moyens (6) d'ajustement de la direction du guide par rapport à la platine (4), qui comprennent un canon (30), qui est pourvu de plusieurs trous traversants (36) rectilignes, l'un desdits trous formant le guide directionnel pour l'organe de perçage, de façonnage ou analogue, **caracterisé en ce que** le canon est adapté pour permettre d'ajuster la direction (C-C) d'application de l'organe de perçage, de façonnage ou analogue, au moins dans deux plans ((X-X, Z-Z), (Y-Y, Z-Z)) sensiblement perpendiculaires l'un à l'autre.

2. Ancillaire suivant la revendication 1, **caractérisé en ce que** le canon (30) comporte un corps tubulaire (32) fermé à une de ses extrémités par un fond (34), les trous traversants (36) étant formés dans le fond et débouchant à l'intérieur du corps tubulaire, lequel corps tubulaire (32) présente en coupe transversale un profil extérieur adapté pour coopérer avec l'ouverture traversante (22) de la platine (4) de façon à immobiliser le canon (30) dans cette ouverture.

3. Ancillaire suivant l'une des revendications 1 ou 2, **caractérisé en ce que** les moyens (6) d'ajustement de la direction du guide sont adaptés pour être reçus et immobilisés dans une ouverture traversante (22) formée dans la platine (4) et débouchant sur la surface d'appui (12) de la platine.

4. Ancillaire suivant l'une quelconques des revendications précédentes, **caractérisé en ce que** les trous (36) des moyens d'ajustement (6, 30, 36) sont ménagés dans le canon (30) au moins suivant lesdits deux plans ((X-X, Z-Z), (X-X, Z-Z)) sensiblement perpendiculaires l'un à l'autre pour permettre l'ajustement de ladite direction d'application (C-C).

5. Ancillaire suivant l'une quelconque des revendications précédentes, **caractérisé en ce que** le canon (30) est adapté pour être relié de façon amovible à la platine (4) pour permettre l'ajustement de ladite direction d'application (C-C) suivant lesdits deux plans ((X-X, Z-Z), (X-X, Z-Z)) sensiblement perpendiculaires l'un à l'autre.

6. Ancillaire suivant l'une quelconque des revendications précédentes, **caractérisé en ce que** les axes des trous (36) sont sensiblement concourants en un point (P) situé sensiblement dans le prolongement géométrique de la face convexe (12) de la platine (4).

7. Ancillaire suivant l'une quelconque des revendications précédentes, **caractérisé en ce qu'**une première série (38) de trous (36) du canon (30) est alignée selon un premier plan (X-X, Z-Z) et une seconde série (40) d'autres trous du canon est alignée suivant un second plan (Y-Y, Z-Z) sensiblement orthogonal au premier plan.

8. Ancillaire suivant la revendication 7, **caractérisé en ce que**, dans chacune des première et seconde séries de trous, les axes des trous (36) situés d'un même côté de l'axe central (Z-Z) du canon (30) sont décalés angulairement les uns par rapport aux autres d'une valeur sensiblement constante.

9. Ancillaire suivant l'une des revendications 7 ou 8, **caractérisé en ce que** d'autres séries (42, 44, 46, 48) de trous (36) sont prévus dans les quadrants définis par les première (38) et seconde (40) séries de trous.

## Claims

1. Ancillary for installing a glenoid implant, having a plate (4) which delimits a bearing surface (12) intended to bear against the glenoid of a scapula (2) of a patient and which forms a directional guide for a drilling, shaping or similar member, the ancillary further having means (6) for adjusting the direction of the guide relative to the plate (4), which comprise a barrel (30), which is provided with several rectilinear through holes (36), one of the said holes forming the directional guide for the drilling, shaping or similar member, **characterised in that** the barrel is adapted to permit adjustment of the direction (C-C) of application of the drilling, shaping or similar member, at least in two planes ((X-X, Z-Z), (Y-Y, Z-Z)) substantially perpendicular to one another.

2. Ancillary according to Claim 1, **characterised in that** the barrel (30) has a tubular body (32) closed at one of its ends by a bottom (34), the through holes (36) being formed in the bottom and opening into the interior of the tubular body, which tubular body (32) has in cross-section an exterior profile adapted to cooperate with the through opening (22) of the plate (4) so as to immobilise the barrel (30) in this opening.

3. Ancillary according to one of Claims 1 and 2, **characterised in that** the means (6) for adjusting the direction of the guide are adapted to be received and immobilised in a through opening (22) formed in the plate (4) and opening onto the bearing surface (12) of the plate.

4. Ancillary according to any one of the preceding claims, **characterised in that** the holes (36) of the adjusting means (6, 30, 36) are made in the barrel (30) at least in the said two planes ((X-X, Z-Z), (X-X, Z-Z)) substantially perpendicular to one another to permit adjustment of the said application direction (C-C).

5. Ancillary according to any one of the preceding claims, **characterised in that** the barrel (30) is adapted to be connected removably to the plate (4) to permit adjustment of the said application direction (C-C) in the said two planes ((X-X, Z-Z), (X-X, Z-Z)) substantially perpendicular to one another.

6. Ancillary according to any one of the preceding claims, **characterised in that** the axes of the holes (36) are substantially concurrent at a point (P) situated substantially in the geometrical extension of the convex face (12) of the plate (4).

7. Ancillary according to any one of the preceding claims, **characterised in that** a first series (38) of holes (36) of the barrel (30) is aligned in a first plane (X-X, Z-Z) and a second series (40) of other holes of the barrel is aligned in a second plane (Y-Y, Z-Z) substantially orthogonal to the first plane.

8. Ancillary according to Claim 7, **characterised in that**, in each of the first and second series of holes, the axes of the holes (36) situated on a same side of the central axis (Z-Z) of the barrel (30) are angularly offset relative to one another by a substantially constant value.

9. Ancillary according to one of Claims 7 and 8, **characterised in that** other series (42, 44, 46, 48) of holes (36) are provided in the quadrants defined by the first (38) and second (40) series of holes.

## Patentansprüche

1. Hilfsvorrichtung zum Einsetzen eines Gelenkimplantats, die eine Platte (4) aufweist, welche eine Stützfläche (12) begrenzt, die dazu bestimmt ist, sich an der Gelenkpfanne eines Schulterblattes (2) eines Patienten abzustützen, und welche eine Richtungsführung für ein Bohr-, Bearbeitungs- oder analoges Teil bildet, wobei die Hilfsvorrichtung außerdem Mittel (6) zur Einstellung der Ausrichtung der Führung in Bezug auf die Platte (4) aufweist, welche eine Hülse (30) aufweisen, die mit mehreren geradlinigen Durchgangsbohrungen (36) versehen ist, wobei eine der Bohrungen die Richtungsführung für das Bohr-, Bearbeitungs- oder analoge Teil bildet, **dadurch gekennzeichnet, dass** die Hülse (130) dafür ausgelegt ist, die Einstellung der Andruckrichtung (C-C) des Bohr-, Bearbeitungs- oder analogen Teils mindestens in zwei Ebenen ((X-X, Z-Z), (Y-Y, Z-Z)), die im Wesentlichen zueinander senkrecht sind, zu ermöglichen.

2. Hilfsvorrichtung nach Anspruch 1, **dadurch gekennzeichnet, dass** die Hülse (30) einen rohrförmigen Schaft (32) aufweist, der an einem seiner Enden durch einen Boden (34) verschlossen ist, wobei die Durchgangsbohrungen (36) in dem Boden (34) ausgebildet sind und im Inneren des rohrförmigen Schaftes (36) münden, wobei das rohrförmige Gehäuse (32) im Querschnitt ein äußeres Profil aufweist, das dafür ausgelegt ist, mit der Durchgangsöffnung (22) der Platte (4) derart zusammenzuwirken, dass die Hülse (30) in dieser Öffnung fixiert wird.

3. Hilfsvorrichtung nach einem der Ansprüche 1 oder 2, **dadurch gekennzeichnet, dass** die Mittel (6) zur Einstellung der Richtung der Führung dafür ausgelegt sind, in einer Durchgangsöffnung (22) aufgenommen und fixiert zu werden, die in der Platte (4) ausgebildet ist und auf der Stützfläche (12) der Platte (4) mündet.

4. Hilfsvorrichtung nach einem der vorhergehenden Ansprüche, **dadurch gekennzeichnet, dass** die Bohrungen (36) der Einstellmittel (6, 30, 36) in der Hülse (30) mindestens entlang der zwei Ebenen ((X-X, Z-Z), (Y-Y, Z-Z)), die im Wesentlichen zueinander senkrecht sind, ausgebildet sind, um die Einstellung der Andruckrichtung (C-C) zu ermöglichen.

5. Hilfsvorrichtung nach einem der vorhergehenden Ansprüche, **dadurch gekennzeichnet, dass** die Hülse (30) dazu ausgelegt ist, lösbar mit der Platte (4) verbunden zu werden, um die Einstellung der Andruckrichtung (C-C) entlang der zwei Ebenen ((X-X, Z-Z), (Y-Y, Z-Z)), die im Wesentlichen zueinander senkrecht sind, zu ermöglichen.

6. Hilfsvorrichtung nach einem der vorhergehenden Ansprüche, **dadurch gekennzeichnet, dass** die Achsen der Bohrungen (36) im Wesentlichen in einem Punkt (P) zusammenlaufen, der sich im Wesentlichen in der geometrischen Verlängerung der konvexen Seite (12) der Platte (4) befindet.

7. Hilfsvorrichtung nach einem der vorhergehenden Ansprüche, **dadurch gekennzeichnet, dass** eine erste Reihe (38) von Bohrungen (36) der Hülse (30) entlang einer ersten Ebene (X-X, Z-Z) ausgerichtet ist und eine zweite Reihe (40) von anderen Bohrungen der Hülse entlang einer zweiten Ebene (Y-Y, Z-Z) ausgerichtet ist, die im Wesentlichen zu der ersten Ebene orthogonal ist.

8. Hilfsvorrichtung nach Anspruch 7, **dadurch gekennzeichnet, dass** sowohl in der ersten als auch in der zweiten Reihe von Bohrungen die Achsen der Bohrungen (36), die sich auf ein und derselben Seite der zentralen Achse (Z-Z) der Hülse (30) befinden, zueinander um einen im Wesentlichen konstanten Wert winkelversetzt sind.

9. Hilfsvorrichtung nach einem der Ansprüche 7 oder 8, **dadurch gekennzeichnet, dass** weitere Reihen (42, 44, 46, 48) von Bohrungen (36) in den Quadranten vorgesehen sind, die durch die erste (38) und zweite (40) Reihe von Bohrungen definiert sind.
